(19) Europäisches Patentamt
European Patent Office
Office européen des brevets

(11) Veröffentlichungsnummer: **0 044 443**
**B1**

# (12) EUROPÄISCHE PATENTSCHRIFT

(45) Veröffentlichungstag der Patentschrift: **01.02.84**

(51) Int. Cl.³: **C 07 D 277/60,** A 61 K 31/425

(21) Anmeldenummer: **81105026.9**

(22) Anmeldetag: **29.06.81**

(54) **Cycloalkano-thiazolyloxamidsäure-Derivate, Verfahren zu ihrer Herstellung und diese enthaltende therapeutische Mittel.**

(30) Priorität: **19.07.80 DE 3027528**

(43) Veröffentlichungstag der Anmeldung: **27.01.82 Patentblatt 82/4**

(45) Bekanntmachung des Hinweises auf die Patenterteilung: **01.02.84 Patentblatt 84/5**

(84) Benannte Vertragsstaaten: **AT BE CH DE FR GB IT LI LU NL SE**

(56) Entgegenhaltungen:
**CH - A - 460 017**
**DE - A - 2 136 233**
**DE - A - 2 413 966**
**DE - A - 2 751 441**
**DE - A - 2 828 091**

(73) Patentinhaber: **BASF Aktiengesellschaft, Carl-Bosch-Strasse 38, D-6700 Ludwigshafen (DE)**

(72) Erfinder: **Eilingsfeld, Heinz, Dr., Pierstrasse 9 A, D-6710 Frankenthal (DE)**
Erfinder: **Neumann, Peter, Dr., Franz-Schubert-Strasse 1, D-6908 Wiesloch (DE)**
Erfinder: **Seybold, Guenther, Dr., Friedrich-Ebert-Strasse 14, D-6701 Neuhofen (DE)**
Erfinder: **Lenke, Dieter, Dr., Kekuleplatz 1, D-6700 Ludwigshafen (DE)**
Erfinder: **Friedrich, Ludwig, Dr., Nibelungenstrasse 8A, D-6831 Bruehl (DE)**

**EP 0 044 443 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Cycloalkano-thiazolyloxamidsäure-Derivate, Verfahren zu ihrer Herstellung und diese enthaltende therapeutische Mittel

Die Erfindung betrifft Cycloalkano-thiazolyloxamidsäuren und ihre Ester, Verfahren zu ihrer Herstellung und diese enthaltende therapeutische Mittel, die bei der Behandlung von allergischen Erkrankungen verwendet werden können.

Es sind eine Reihe von Derivaten der Oxamidsäure und ihrer Ester bekannt, beispielsweise aus der DE-OS 2 413 966 Aryl- und Hetaryldervate, der DE-OS 2 828 091 und der veröffentlichten europäischen Patentanmeldung 0 006 368 Thiazolderivate und aus der DE-OS 2 751 441 und 2 656 468 Benzothiazolderivate, für die eine Verwendung bei der Bekämpfung oder Unterdrückung von allergischen Reaktionen beschrieben wird. Ihre Wirkungen befriedigen jedoch nicht immer.

Es wurde nun gefunden, daß Verbindungen der allgemeinen Formel (I)

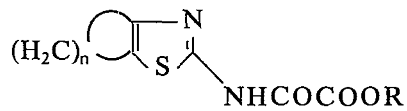

(I)

in der n eine ganze Zahl von 2 bis 10 und R ein Wasserstoffatom, gegebenenfalls ersetzt durch ein physiologisch verträgliches Metall- oder Ammoniumkation, einen Alkylrest mit 1 bis 4 Kohlenstoffatomen oder einen Rest der Formel

$$—CH_2CH_2O—CH_2CH_3$$

bedeuten, wertvolle pharmakologische Eigenschaften, insbesondere als Antiallergika, aufweisen.

Neben den in den Ausführungsbeispielen angegebenen Verbindungen sollen als erfindungsgemäße Verbindungen im einzelnen noch genannt werden:

N-(Cyclooctano[1,2-d]thiazol-2-yl)-oxamidsäure
und ihr Methyl-, Ethyl- und 2-Ethoxyethylester,
N-(Cyclononano [1,2-d]thiazol-2-yl)-oxamidsäure
und ihr Methyl-, Ethyl- und 2-Ethoxyethylester,
N-(Cyclodecano[1,2-d]thiazol-2-yl)-oxamidsäure
und ihr Methyl-, Ethyl- und 2-Ethoxyethylester
N-(Cyclododecano[1,2-d]thiazol-2-yl)-oxamidsäure
und ihr Methyl-, Ethyl und 2-Ethoxyethylester.

Im Falle einer Oxamidsäure der allgemeinen Formel (I), wenn R ein Wasserstoffatom bedeutet, kommen als physiologisch verträgliche Metall- oder Ammonium-Kationen beispielsweise in Betracht die der Alkali-Metalle, wie Lithium, Natrium und Kalium, der Erdalkali-Metalle, wie Magnesium und Calcium, sowie des Aluminiums, Eisens oder des Ammoniums und geeigneter primärer, sekundärer und tertiärer Amine, wie Methylamin, Dimethylamin, Triäthylamin, Äthylamin, Dibutylamin, Triisopropylamin, N-Methylhexylamin, Cyclopentylamin, Benzylamin, Phenyläthylamin, Äthylendiamin, Diäthylentriamin, heterocyclische Amine, wie Piperidin, Morpholin, Pyrrolidin, Piperazin und deren niedere Alkyl-Derivate, wie 1-Methylpiperidin, 4-Äthylmorpholin, 1-Isopropylpyrrolidin, 2-Methylpyrrolidin, 1,4-Dimethylpiperazin, 2-Methylpiperazin, 1,4-Dimethylpiperazin oder 2-Methylpiperazin, Amine, die zusätzlich wasserlösliche oder hydrophile Gruppen enthalten, wie z. B. Mono-, Di- und Triäthanolamin, Äthyldiäthanolamin, N-Butyläthanolamin, 2-Amino-1-butanol, 2-Amino-2-äthyl-1,3-propandiol, 2-Amino-2-methyl-1-propanol, Tris-(hydroxymethyl)-aminomethan, N-Phenyläthanolamin. Zu den Aminkationen gehören auch quaternäre Ammoniumverbindungen, wie Tetramethylammonium-, Tetraäthylammonium-, Benzyltrimethylammonium- und Phenyltriäthylammonium-Kationen.

Von den Verbindungen der allgemeinen Formel (I) sind die bevorzugt, in denen

n eine ganze Zahl von 3 bis 8
R ein Wasserstoffatom, einen geradkettigen oder verzweigten Alkylrest mit 1 bis 4 Kohlenstoffatomen oder 2-Ethoxyethyl darstellt, wobei ein Wasserstoffatom für R durch Natrium, Kalium, ein niederes Alkyl-, Dialkyl- oder Trialkylammonium, Ethanolammonium oder Tris-(hydroxymethyl)-methylammonium ersetzt sein kann.

Besonders bevorzugt sind Verbindungen der allgemeinen Formel (I), in denen n eine ganze Zahl von 3 bis 5 darstellt und R die oben angegebenen bevorzugten Bedeutungen hat.

Die erfindungsgemäßen Verbindungen der allgemeinen Formel (I) werden hergestellt, indem man eine Verbindung der allgemeinen Formel (II)

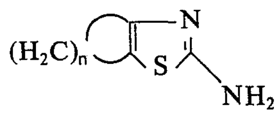

(II)

in der n die für die allgemeine Formel (I) angegebenen Bedeutungen hat, mit einem Oxalsäureesterhalogenid der allgemeinen Formel (III)

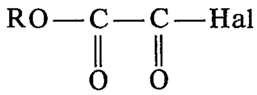

(III)

in der R außer Wasserstoff die für die allgemeine Formel (I) angegebenen Bedeutungen hat und Hal ein Halogenatom, bevorzugt ein Chloratom, bedeuten, in einem Lösungsmittel und in Anwesenheit eines Säureakzeptors umsetzt und gegebenenfalls die erhaltene Verbindung zur Säure verseift und in ein physiologisch verträgliches Salz mit einem Metall- oder Ammoniumkation überführt.

Geeignete Lösungsmittel sind beispielsweise Methylenchlorid, Chloroform, Tetrachlorkohlenstoff, 1,2-Dichloräthan, 1,1,1-Trichloräthan, Essigsäureäthylester, Dimethylformamid, Dimethylacetamid, N-Methylpyrrolidon, Tetrahydrothiophendioxid, Hexamethylphosphorsäuretriamid, Tetrahydrofuran, Dioxan oder Mischungen derselben. Beispiele für Säureakzeptoren sind Triäthylamin, Tributylamin, Pyridin. Die Reaktion zwischen den Aminothiazolen (II) und den Oxalsäureesterhalogeniden wird vorzugsweise bei relativ niederen Temperaturen durchgeführt, wobei Temperaturen zwischen 0°C und 30°C üblich sind. An Stelle der freien Aminothiazole kann man die Hydrochloride verwenden, wenn man ein zusätzliches Äquivalent Base einsetzt.

Niedere Alkylester mit 1 bis 4 C-Atomen der allgemeinen Formel (I) können auch hergestellt werden durch Umsetzung einer Verbindung der allgemeinen Formel (II) mit einem Oxalsäuredialkylester. Die Umsetzung eines 2-Aminothiazols der allgemeinen Formel (II) mit einem Oxalsäuredialkylester wird in unverdünnter Form direkt oder unter Zusatz eines Lösungsmittels, wie Xylol, bei einer Temperatur zwischen Raumtemperatur und Rückflußtemperatur des Reaktionsgemisches durchgeführt.

Durch Hydrolyse der erhaltenen erfindungsgemäßen Oxamidsäureester, insbesondere der Oxamidsäureethylester, mit einer wäßrigen oder wäßrig/alkoholischen Lösung einer Base, wie Natriumhydroxid, Kaliumhydroxid, Kaliumcarbonat oder auch Kaliumacetat, bei Temperaturen zwischen Raumtemperatur und etwa 100°C erhält man die entsprechenden Alkalimetalloxamate. Die Behandlung der Salze mit wäßriger Säure, wie z. B. Salzsäure oder Schwefelsäure, liefert die Oxamidsäure der allgemeinen Formel (I) (R=H).

Die Oxamidsäuren lassen sich in üblicher Weise in Metall- oder Ammoniumsalze umwandeln.

Weiter können sie nach an sich bekannten Methoden in Oxamidsäureester überführt werden, beispielsweise durch Veresterung in Gegenwart eines sauren Katalysators oder eines Carbodiimids, wie es in Chem. Ber. 100, 16 (1967) bzw. in Acta Chem. Scand. B 33, 410 (1979) beschrieben ist. Die Veresterung der freien Oxamidsäure kann außerdem mittels eines Dimethylformamid-Acetals nach der Imidazolid-Methode [Chem. Ber. 95, 1284 (1962)] oder nach dem in Bull. Chem. Soc. Japan 50, 1863 (1977) beschriebenen Verfahren erfolgen.

Die als Ausgangsstoffe verwendeten, in 4,5-Stellung überbrückten 2-Aminothiazole der allgemeinen Formel (II) sind teilweise in der Literatur beschrieben, z. B. in »The Chemistry of Heterocyclic Compounds, Thiazoles and its Derivatives«, Hg. J. v. Metzger, Band 1. Neue Verbindungen dieses Typs können nach den dort beschriebenen Ringschlußmethoden dargestellt werden.

Die erfindungsgemäßen Verbindungen weisen antiallergische Eigenschaften, aufgrund deren sie als wertvolle Heilmittel bei der Behandlung von allergischen Erkrankungen des Respirations-, des Gastro-Intestinaltraktes und der Haut, beispielsweise allergischen Asthmas, allergischer Rhinitis oder Nahrungsmittel-Allergien, verwendet werden können, auf. Gegenüber dem bekannten Antiallergikum Cromolyne zeichnen sich die erfindungsgemäßen Verbindungen im Tierexperiment bei der passiven cutanen Anaphylaxie der Ratte dadurch aus, daß sie oral wirksam sind und zusätzlich über eine wesentlich längere Wirkdauer verfügen.

Die antiallergische Wirkung wurde an Ratten getestet. Zur Prüfung wurde das Modell der passiven cutanen Anaphylaxie (PCA) verwendet.

Narkotisierte männliche Ratten (100 bis 140 g) werden durch intradermale Injektion (rasierte Rückenhaut) von 0,1 ml eines Ovalbumin-Antiserums sensibilisiert. Nach einer Sensibilisierungsperiode von ca. 48 Stunden erfolgt die Behandlung (intraperitoneale bzw. orale Applikation) mit unterschiedlichen Dosierungen (10 Tiere/Dosis) der Prüfsubstanzen. 15 bis 20 Minuten nach der Behandlung wird eine Antigen/Evansblau-Mischung (10 mg/kg Ovalbumin in 2%iger Evansblau-Lösung) den Versuchstieren intravenös injiziert. 30 Minuten später werden die Tiere getötet, die Rückenhaut abgezogen und auf der inneren Oberfläche der Durchmesser der kreisförmigen Blaufärbung gemessen. Die Größe der Farbflecke unbehandelter Kontrolltiere ist standardisierbar. Antiallergisch wirksame Substanzen verkleinern dosisabhängig den Durchmesser der Farbflecke. Als ED 50 % wird die Dosis angegeben die im Vergleich zu medikamentös unbehandelten Kontrolltieren den Durchmesser der Farbflecke um 50 % herabsetzt.

Außer der antiallergischen Wirkung wurde die akute Toxizität an Gruppen von je 2 NMRI-Mäusen, Gewicht 20 bis 26 g, bei intraperitonealer Applikation und an je 2 Sprangue-Dawley-Ratten, Gewicht 120 bis 150 g, bei oraler Applikation ermittelt. Die Bestimmung der LD 50 erfolgte an Gruppen von je 10 NMRI-Mäusen, Gewicht 20 bis 26 g, nach intraperitonealer Applikation. Die Nachbeobachtungszeit betrug 14 Tage.

Die erfindungsgemäßen Verbindungen sind antiallergisch hochwirksam. Aus Tabelle 1 geht hervor, daß diese Verbindungen nach intraperitonealer Gabe wesentlich wirksamer sind als die Vergleichsverbindung $V_1$ (N-Benzthiazol-2-yl-oxamidsäureethylester, DE-OS 2 751 441, Beispiel 1) und das Handelspräparat Cromolyne.

Tabelle 1

Antiallergische Wirkung nach intraperitonealer Gabe von 1 mg/kg Prüfsubstanz. PCA. Ratte.

| Beispiel | %Hemmung $X \pm SX$ |
|---|---|
| 2 | $61 \pm 8,8$ |
| 4 | $63 \pm 6,6$ |
| 3 | $34 \pm 6,0$ |
| 1 | $25 \pm 4,7$ |
| 5 | $33 \pm 7,9$ |
| 6 | $73 \pm 7,5$ |
| $V_1$ | $26 \pm 6,5$ |
| Cromolyne | $21 \pm 3,6$ |

Die Überprüfung der antiallergischen Wirkung nach oraler Applikation ergab (s. Tab. 2), daß die neuen Verbindungen 2,8 (Beispiel 4) bis 11,9 (Beispiel 2) mal wirksamer sind als die Vergleichsverbindung $V_1$. Die Handelssubstanz Cromolyne ist in dieser Versuchsanordnung bis 100 mg/kg p. o. unwirksam.

Tabelle 2

Antiallergische Wirkung (orale Gabe) und akute Toxizität

| Beispiel Nr. | Hemmung der PCA Ratte | | LD 50 mg/kg | |
|---|---|---|---|---|
| | ED 50% mg/kg | R.W.[1]) | Maus i. p. | Ratte p. o. |
| 2 | 0,722 | 11,90 | 422 | >2 150 |
| 1 | 1,59 | 5,40 | ca. 464 | >2 150 |
| 5 | 2,35 | 3,65 | – | >2 150 |
| 4 | 3,09 | 2,78 | ca. 464 | >2 150 |

Fortsetzung

| Beispiel Nr. | Hemmung der PCA Ratte | | LD 50 mg/kg | |
|---|---|---|---|---|
| | ED 50% mg/kg | R.W.[1]) | Maus i.p. | Ratte p.o. |
| $V_1$ | 8,60 | 1,00 | ca. 464 | >2 150 |
| Cromolyne | 100 | – | – | >2 150 |

[1]) Relative Wirksamkeit.

Aufgrund der besseren Wirksamkeit ergibt sich ein wesentlich größerer Abstand zwischen den toxischen und den wirksamen Dosen.

Für die therapeutische Anwendung werden Einzeldosen von 0,1 bis 100 mg verwendet.

Gegenstand der vorliegenden Erfindung sind demnach auch therapeutische Mittel oder Zubereitungen, die neben üblichen Träger- und Verdünnungsmitteln eine Verbindung der Formel I als Wirkstoff enthalten.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Weiterhin kommen Inhalate und parenterale Zubereitungen, wie Injektionslösungen, in Betracht.

Die galenischen Applikationsformen, fest oder flüssig, werden in üblicher Weise hergestellt. Die Wirkstoffe können dabei mit den gebräuchlichen galenischen Hilfsmitteln, wie Talkum, Gummiarabikum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wäßrigen oder nicht wäßrigen Trägern, Netzmitteln, Dispergiermitteln, Emulgatoren und/oder Konservierungsmitteln, verarbeitet werden (vgl. L. G. Goodman, A. Gilman, The Pharmacological Basis of Therapeutics).

## Beispiel 1

### N-(Cyclopentano[1,2-d]thiazol-2-yl)-oxamidsäureethylester

Zu einer Lösung von 6,85 g 2-Amino-cyclopentano[1,2-d]thiazol in 125 ml trockenem Methylenchlorid und 8,05 ml trockenem Pyridin tropft man bei etwa 10°C eine Lösung von 6,5 ml Ethyloxalylchlorid in 15 ml Methylenchlorid. Man rührt noch 1 Stunde bei 10°C und 2 Stunden bei Raumtemperatur nach. Man filtriert, engt das Filtrat ein und behandelt den Rückstand mit 0,5 n Salzsäure. Der feste Rückstand wird abgetrennt, mit Wasser gewaschen und getrocknet. Nach säulenchromatographischer Reinigung (Kieselgel/Methylenchlorid) erhält man 1,8 g der Titelverbindung vom Schmp. 98–100°C.

## Beispiel 2

### N-(Cyclohexano[1,2-d]thiazol-2-yl)-oxamidsäureethylester

Zu einer Lösung von 30,4 g 2-Amino-cyclohexano[1,2-d]thiazol in 120 ml trockenem Dimethylformamid, die 24 g Triethylamin enthält, tropft man bei 5 bis 15°C 32,4 g Ethyloxalylchlorid. Man rührt noch 1 Stunde bei einer Temperatur von 10°C und danach etwa 20 Stunden bei Raumtemperatur. Man gießt auf Eiswasser, rührt das Gemisch ca. 15 Minuten, saugt den ausgefallenen Niederschlag ab und trocknet im Vakuum. Man behandelt die Substanz mit warmem Butanol, filtriert und engt das Filtrat im Vakuum teilweise ein. Man erhält 20 g der Titelverbindung vom Schmp. 120°C.

$C_{11}H_{14}O_3N_2S$ (254,3)

Ber.: C 51,95, H 5,55, N 11,02, S 12,61,
Gef.: C 52,3, H 5,5, N 11,2, S 12,7.

Beispiel 3

N-(Cycloheptano[1,2-d]thiazol-2-yl)-oxamidsäureethylester

Aus 7,8 g 2-Amino-cycloheptano[1,2-d]thiazol erhält man analog Beispiel 2 nach Kristallisation aus Ethanol 8,5 g der Titelverbindung vom Schmp. 162—163°C.

$C_{12}H_{16}O_3N_2S$ (263,3)

Ber.: C 53,71, H 6,01, N 10,44, S 11,95,
Gef.: C 53,8, H 6,2, N 10,4, S 12,4.

Beispiel 4

N-(Cyclohexano[1,2-d]thiazol-2-yl)-oxamidsäure

25,4 g N-(Cyclohexano[1,2-d]thiazol-2-yl)-oxamidsäureethylester werden in einer Lösung von 10 g Kaliumacetat in 200 ml Wasser suspendiert und 3 Stunden unter Rückfluß erhitzt. Man saugt heiß ab und läßt das Filtrat erkalten. Die ausgeschiedenen Kristalle werden abgesaugt und getrocknet. 5 g des so erhaltenen Kaliumsalzes werden in 500 ml Wasser 3 Stunden gerührt. Man filtriert und säuert das Filtrat mit 0,5 n Salzsäure an. Der entstandene Niederschlag wird abgesaugt, mit Wasser gewaschen und getrocknet. Man erhält 2 g der Titelverbindung vom Schmp. 235—240°C (Zers.).

$C_9H_{10}O_3N_2S$ (226,2)

Ber.: C 47,78, H 4,46, N 12,38, S 14,17,
Gef.: C 47,7, H 4,4, N 12,5, S 14,1.

Beispiel 5

N-(Cyclohexano[1,2-d]thiazol-2-yl)-oxamidsäure-tert.-butylester

Aus 7,6 g 2-Amino-cyclohexano[1,2-d]thiazol erhält man analog Beispiel 2 mit tert.-Butyloxalylchlorid nach Umkristallisation aus wäßrigem Äthanol/Aktivkohle und anschließendem Umfällen aus N-Methylpyrrolidon/verd. Ammoniaklösung 1,9 g der Titelverbindung vom Schmp. 125—127°C.

$C_{13}H_{18}N_2O_3S$ (282,3)

Ber.: C 55,3, H 6,43, N 9,92, S 11,36,
Gef.: C 55,4, H 6,4, N 9,9, S 11,2.

Beispiel 6

N-(Cyclohexano[1,2-d]thiazol-2-yl)-oxamidsäure-ethanolaminsalz

Zu einer Lösung von 6,78 g N-(Cyclohexano[1,2-d]thiazol-2-yl)-oxamidsäure in 70 ml heißem Methanol gibt man eine heiße Lösung von 1,8 g Ethanolamin in 20 ml Methanol. Man erhitzt etwa 2 Stunden unter Rückfluß bewahrt anschließend die Reaktionsmischung im Kühlschrank auf. Die nach mehrtägigem Stehen erhaltenen Kristalle werden abgesaugt, mit Ligroin gewaschen und getrocknet. Man isoliert 3,4 g der (gut wasserlöslichen) Titelverbindung vom Schmp. 120 bis 123°C.

$C_{11}H_{17}N_3O_4S$ (287)

Ber.: C 45,98, H 5,96, N 14,62, S 11,16,
Gef.: C 46,1, H 6,0, N 13,8, S 10,6.

Beispiel 7

N-(Cyclohexano[1,2-d]thiazol-2-yl)-oxamidsäure-2-ethoxyethylester

Zu einer Lösung von 7,7 g 2-Amino-cyclohexano-[1,2-d]-thiazol in 50 ml trockenem Methylenchlorid und 8,05 ml trockenem Pyridin tropft man unterhalb von 5°C 9,9 g frisch destilliertes Oxalsäure-2-ethoxyethylesterchlorid. Man rührt noch 3 Stunden bei 0 bis 5°C nach. Anschließend wird abfiltriert, das Filtrat unter vermindertem Druck bei 20°C eingeengt und der ölige Rückstand mit 0,5 n Salzsäure behandelt. Der kristalline Niederschlag wird nach dem Trocknen in Ethanol aufgenommen und mit Aktivkohle behandelt. Nach der Filtration wird unter vermindertem Druck eingeengt, der Rückstand mit wenig Ethanol verrührt, abgesaugt und getrocknet. Man erhält 3,2 g der Titelverbindung vom Schmp. 78 bis 79°C.

$C_{13}H_{18}N_2O_4S$ (298)

| | | | | |
|---|---|---|---|---|
| Ber.: | C 52,37, | H 6,08, | N 9,39, | S 10,75, |
| Gef.: | C 52,6, | H 5,9, | N 9,6, | S 11,0. |

Beispiele für pharmazeutische Zubereitungen

Tabletten

| | | |
|---|---|---|
| a) | Wirkstoff | 0,100 g |
| | Stearinsäure | 0,010 g |
| | Traubenzucker | 1,890 g |
| | | 2,000 g |
| b) | Wirkstoff | 0,020 g |
| | Stearinsäure | 0,020 g |
| | Traubenzucker | 1,960 g |
| | | 2,000 g |

Die Bestandteile werden in üblicher Weise zu Tabletten der vorstehend angegebenen Zusammensetzung verarbeitet.

Inhalationsaerosol

| | |
|---|---|
| Wirkstoff | 1,00 Teile |
| Sojalecithin | 0,20 Teile |
| Treibgasmischung (®Frigen 11, 12 und 114) ad | 100,00 Teile |

Die Zubereitung wird vorzugsweise in Aerosolbehälter mit Dosierventil abgefüllt. Der einzelne Hub wird so bemessen, daß eine Dosis von 5 bis 20 mg Wirkstoff abgegeben wird.

Ampullen (Injektionslösungen)

| | |
|---|---|
| Wirkstoff | 50,0 Gew.-Teile |
| Natriumpyrosulfit | 1,0 Gew.-Teil |
| Dinatriumsalz der Äthylendiamintetraessigsäure | 0,5 Gew.-Teile |
| Natriumchlorid | 8,5 Gew.-Teile |
| doppelt destilliertes Wasser ad | 1000,0 Gew.-Teile |

Der Wirkstoff und die Hilfsstoffe werden in einer ausreichenden Menge Wasser gelöst und mit der notwendigen Menge Wasser auf die gewünschte Konzentration gebracht. Die Lösung wird filtriert und unter aseptischen Bedingungen in 1 ml Ampullen abgefüllt. Zuletzt werden die Ampullen sterilisiert und verschlossen. Jede Ampulle enthält 50 mg Wirkstoff.

**Patentansprüche**

1. Verbindungen der allgemeinen Formel (I)

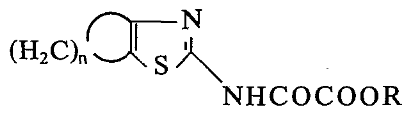

(I)

in der n eine ganze Zahl von 2 bis 10 und R ein Wasserstoffatom, gegebenenfalls ersetzt durch ein physiologisch verträgliches Metall- oder Ammoniumkation, einen Alkylrest mit 1 bis 4 Kohlenstoffatomen oder einen Rest der Formel

$$—CH_2CH_2O—CH_2CH_3$$

bedeuten.

2. Verbindungen der allgemeinen Formel (I), in denen n eine ganze Zahl von 3 bis 5 bedeutet.

3. N-(Cyclohexano[1,2-d]thiazol-2-yl)-oxamidsäure, ihre physiologisch verträglichen Metall- oder Ammonium-Salze und ihr Ethylester.

4. Verfahren zur Herstellung von Verbindungen der allgemeinen Formel (I) nach Anspruch 1, dadurch gekennzeichnet, daß man eine Verbindung der allgemeinen Formel (II)

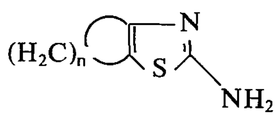

(II)

in der n die für die allgemeine Formel (I) in Anspruch 1 angegebene Bedeutung hat, mit einem Oxalylhalogenid der allgemeinen Formel (III)

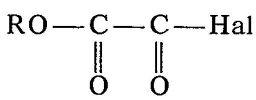

(III)

in der R außer Wasserstoff die für die allgemeine Formel (I) in Anspruch 1 angegebenen Bedeutungen hat und Hal ein Halogenatom bedeuten, in einem Lösungsmittel und in Anwesenheit eines Säureakzeptors umsetzt und die erhaltene Verbindung gegebenenfalls zur Säure verseift und in ein physiologisch verträgliches Salz überführt.

5. Therapeutisches Mittel, gekennzeichnet durch einen Gehalt an einer Verbindung der allgemeinen Formel (I) nach Anspruch 1 als Wirkstoff neben üblichen Trägerstoffen und Verdünnungsmitteln.

**Claims**

1. A compound of the general formula (I)

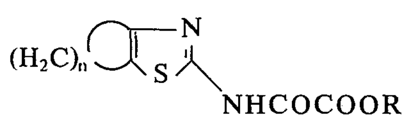

(I)

where n is an integer from 2 to 10, and R is hydrogen which may be replaced by a physiologically tolerated metal cation or amine cation, or is alkyl of 1 to 4 carbon atoms, or is a radical of the formula

$$—CH_2CH_2O—CH_2CH_3$$

2. A compound of the general formula (I), where n is an integer from 3 to 5.

3. N-(Cyclohexano[1,2-d]thiazol-2-yl)-oxamic acid, its physiologically tolerated metal or ammonium salts, and its ethyl esters.

4. A process for the preparation of a compound of the general formula (I) as claimed in claim 1, wherein a compound of the general formula (II)

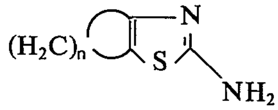

(II)

where n has the meaning given for general formula (I) in claim 1, is reacted with an oxalyl halide of the general formula (III)

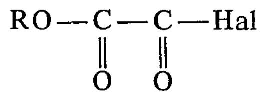

(III)

where R has the meanings given for general formula (I) in claim 1, except for hydrogen, and Hal is halogen, in a solvent and in the presence of an acid acceptor, and, where required, the compound obtained is hydrolyzed to the acid and converted into a physiologically tolerated salt.

5. A therapeutic agent which contains a compound of the general formula (I) as claimed in claim 1 as the active compound, in addition to conventional carriers and diluents.

**Revendications**

1. Composés de la formule générale (I)

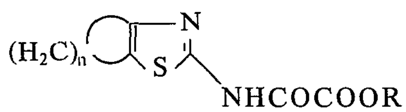

(I)

dans laquelle n est un nombre entier de 2 à 10 et R désigne un atome d'hydrogène éventuellement remplacé par un cation de métal ou d'ammonium physiologiquement compatible, un radical alkyle en $C_1$ à $C_4$ ou un groupe de la formule

$$—CH_2CH_2O—CH_2CH_3$$

2. Composés de la formule générale (I), dans laquelle n est un nombre entier de 3 à 5.

3. L'acide N-(Cyclohexano[1,2-d]thiazol-2-yl)-oxamique, ses sels de métal ou d'ammonium physiologiquement compatibles et son ester éthylique.

4. Procédé de préparation de composés de la formule générale (I) selon la revendication 1, caractérisé en ce que l'on fait réagir, dans un solvant et en présence d'un fixateur d'acide, un composé de la formule générale (II)

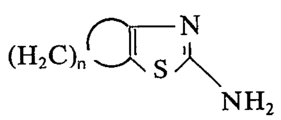

(II)

dans laquelle n possède la signification définie pour la formule générale (I) de la revendication 1, avec un halogénure d'oxalyle de la formule générale (III)

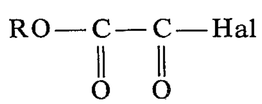

(III)

dans laquelle R possède les significations définies pour la formule générale (I) de la revendication 1, à l'exception de l'atome d'hydrogène, et Hal désigne un atome d'halogène, le composé obtenu pouvant, le cas échéant, être saponifié en acide et transformé en un sel physiologiquement acceptable.

5. Composition thérapeutique, caractérisée en ce qu'elle contient un composé de la formule générale (I) de la revendication 1 comme principe actif à côté d'excipients et de diluants usuels.